# FASCICULE DE BREVET EUROPEEN

(11) **EP 0 658 349 B1**
(45) Date de publication et mention de la délivrance du brevet: **17.11.1999**
(21) Numéro de dépôt: 94420353.8
(22) Date de dépôt: 12.12.1994
(51) Int. Cl.: A61L 2/26

(54) **Appareil stérilisateur de biberons**
Vorrichtung zum Sterilisieren von Babymilchflaschen
Apparatus for sterilizing baby milk bottles

(30) Priorité: 14.12.1993 FR 9315507
(43) Date de publication de la demande: 21.06.1995
(73) Titulaire: HUTCHINSON, 75008 Paris (FR)
(72) Inventeur: ALLEGRE, Jean Paul, 42013 Saint Etienne (FR)
(74) Mandataire: Dupuis, François

(56) Documents cités:
- EP-A- 0 367 559
- GB-A- 2 247 624
- GB-A- 2 262 232
- US-A- 5 213 776

## Description

L'invention se rattache au secteur technique des appareils stérilisateurs de biberons.

Les mamans, pour les enfants en très bas âge, doivent nourrir ces derniers à partir de produits à base de lait. Indépendamment de l'allaitement maternel qui n'est que provisoire, elles utilisent une pluralité de biberons nécessaires à l'absorption de volumes de laitage plusieurs fois dans la journée, selon des cycles bien connus.

Le nettoyage et la stérilisation des biberons sont des contraintes en égard des manipulations à effectuer.

Dans la pratique courante, les biberons sont positionnés dans des réceptacles types casseroles, marmites et similaires et sont stérilisés dans l'eau bouillante. Avec le développement des appareils électroménagers et en particulier des fours à micro-ondes, Il a aussi été proposé des appareils stérilisateurs dans lesquels les biberons sont traités en nombre, ces stérilisateurs étant adaptés pour être introduits dans lesdits fours à micro-ondes. Un appareil stérilisateur de ce type est notamment été décrit dans le document GB-A-2.247.624. Cet appareil comprend un réceptacle de fond susceptible de recevoir

Ce document décrit un appareil pour maintien de biberons en vue de leur stérilisation du type comprenant un réceptacle de fond susceptible de recevoir un volume d'eau, ledit réceptacle recevant, à une hauteur déterminée, une grille monobloc avec une pluralité de découpes formées en croissant de dimensions préétablies et correspondant à un demi-diamètre du col de biberon permettant l'engagement et le positionnement des biberons par leur col dans une position inclinée par rapport à la verticale, ledit réceptacle recevant une cloche d'obturation, l'appareil pouvant être introduit dans un four à micro-ondes, ladite grille permettant le positionnement des biberons, tétines et capuchons.

En pratique, un tel appareil présente certains inconvénients. Compte tenu des conditions de manipulations de l'appareil stérilisateur, il s'avère que les biberons sont très instables pouvant très facilement s'entrechoquer et s'échapper de leur moyen de logement et de tenue en forme de croissant.

Le but recherché selon l'invention était donc de concevoir un appareil stérilisateur obviant aux inconvénients précités en proposant de nouveaux moyens associés à la grille permettant une stabilité accrue des biberons.

Un autre but selon l'invention était de concevoir un appareil stérilisateur approprié pour s'adapter à toutes contraintes dimensionnelles des fours à micro-ondes et de leur hauteur en particulier.

Ces buts et d'autres encore ressortiront bien de la suite de la description.

L'appareil pour maintien de biberons en vue de leur stérilisation selon l'invention est remarquable en ce que le réceptacle présente dans son fond un ou des bandeaux supports de la grille, ladite grille étant munie d'une pluralité desdits moyens permettant l'engagement et le positionnement des biberons, ces moyens étant conformés et agencés pour assurer en même temps la tenue de chaque biberon par son col et son maintien par son corps, chacun desdits moyens étant agencé avec une assise profilée présentant un plan incliné et une incurvation transversale enveloppant partiellement le corps du biberon, cette assise se prolongeant dans sa partie basse avec une forme en collet susceptible d'entourer le col du biberon, ladite forme présentant une ouverture de passage du col du biberon perpendiculaire au plan de l'assise.

Ces caractéristiques et d'autres encore ressortiront bien de la suite de la description.

Pour fixer l'objet de l'invention illustré non limitativement aux figures des dessins où :
La figure 1 est une vue en perspective éclatée avant montage d'un appareil stérilisateur de biberons selon l'invention.
La figure 2 est une vue de dessus, sans couvercle, illustrant la position des différents biberons dans une première réalisation.
La figure 3 est une vue en coupe selon la ligne brisée (A-A) de la figure 2.
La figure 4 est une vue en variante en coupe illustrant les moyens de tenue des biberons.
La figure 5 est une vue en perspective illustrant une variante de positionnement du biberon en ligne à l'intérieur de l'appareil.

Afin de rendre plus concret l'objet de l'invention, on le décrit maintenant d'une manière non limitative aux figures des dessins.

L'appareil stérilisateur selon l'invention est référencé dans son ensemble par (1). Il comprend un réceptacle de fond (2) par exemple de forme circulaire réalisé en tout matériau. Ce réceptacle de hauteur appropriée présente intérieurement et dans sa partie médiane un bandeau support (2.1) formant anneau délimitant un volume de réception d'eau à bouillir. Le volume ainsi défini peut correspondre par exemple à une dose de liquide suffisante pour la stérilisation des biberons.

En variante, un ou plusieurs bandeaux concentriques peuvent le cas échéant être prévus et le fluide est disposé dans tout ou partie du fond du réceptacle. Par son chant supérieur (2.2), le ou les bandeaux permettent le positionnement et la stabilité d'une grille perforée (3) de forme similaire et complémentaire à celle intérieure du réceptacle. Cette grille présente une pluralité de découpes (3.1) débouchant dans la partie de fond du réceptacle et permettant ainsi une diffusion de la vapeur d'eau résultant de l'ébullition de l'eau. La grille (3) est agencée pour permettre le positionnement des biberons (B) en position inclinée, ainsi que celui des tétines (T) et des couvercles (C).

Le réceptacle reçoit le long de sa bordure supérieure périphérique une cloche (4) en tout matériau approprié pouvant s'auto-verrouiller à ce dernier. La cloche présente des découpes (4.1) profilées en L susceptibles de pénétrer dans des ergots (2 - 3) formés intérieurement sur le bandeau supérieur du réceptacle.

Selon l'invention, la grille (3) est aménagée avec une pluralité de moyens (5) conformés et agencés pour permettre, d'une part, la tenue du biberon par et autour de son col et, d'autre part, son maintien par son corps, et dans une position inclinée.

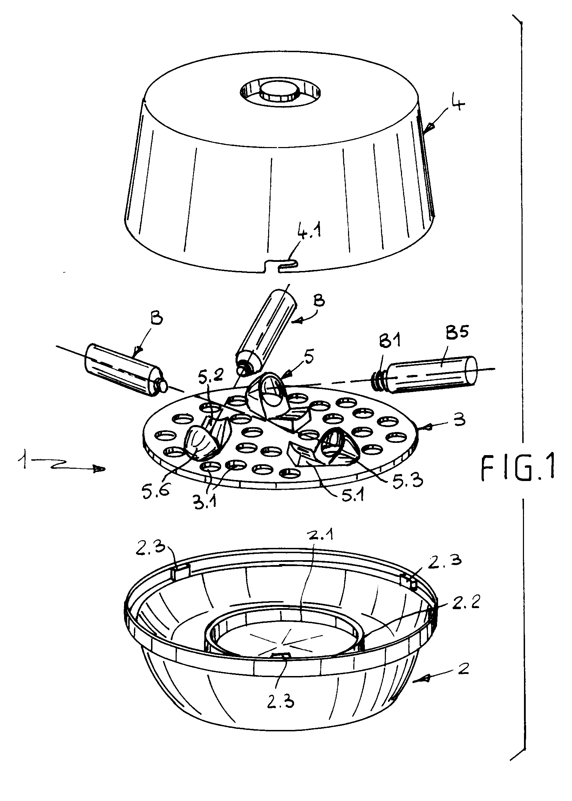

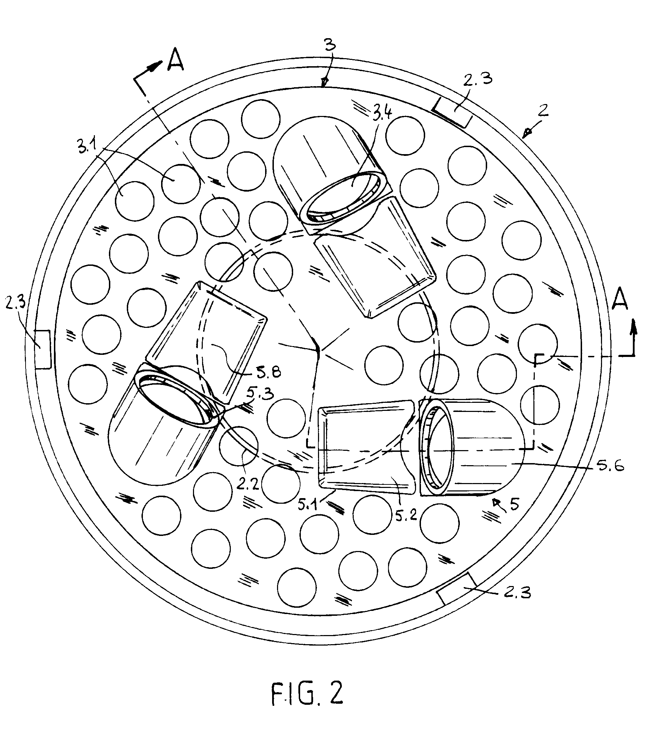

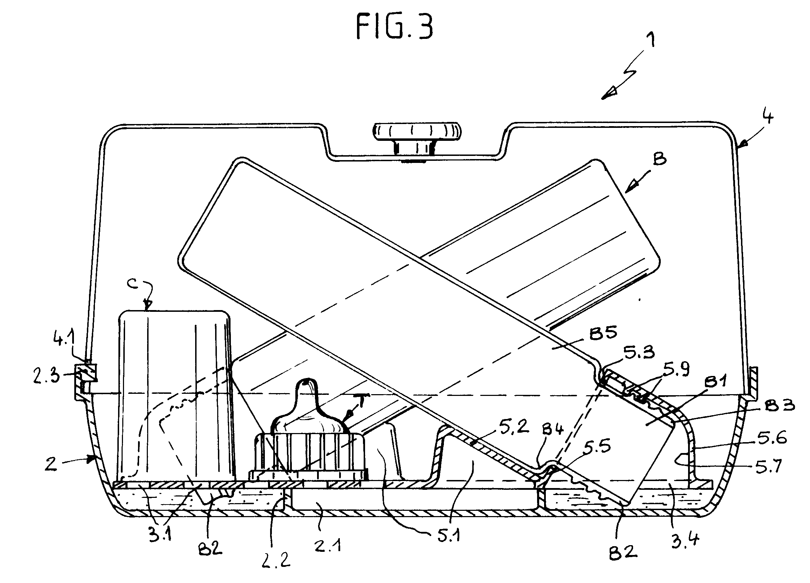

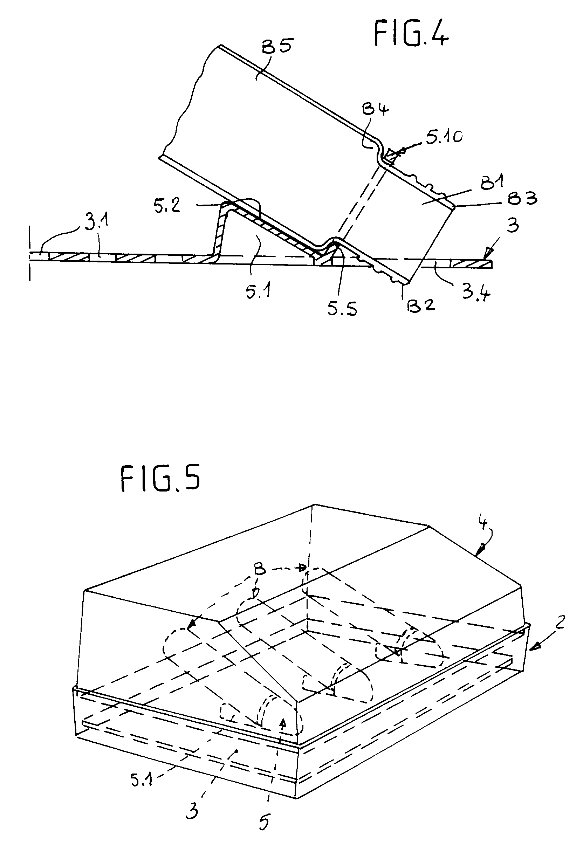

A cet effet, chaque moyen (5) comprend monobloc et directement formée avec la grille, une assise (5.1) de conformation triangulaire avec une base dans le plan horizontal de la grille et avec un plan (5.2) incliné et incurvé permettant le maintien et le guidage du corps du biberon. Cette assise se prolonge dans sa partie basse avec une forme (5.3) en collet susceptible d'entourer le col du biberon en assurant ainsi sa stabilité. Cette forme illustrée aux figures 1 à 4 se présente comme une bouche (5.4) avec une ouverture (5.5) perpendiculaire au plan incliné (5.2) de l'assise et permettant le positionnement du col (B1) du biberon. Elle se prolonge ensuite par une partie pleine (5.6) coudée reliée au plan horizontal de la grille, tandis qu'une découpe (3.4) est formée sur la grille à l'intérieur de la bouche pour permettre l'accès dans la partie de fond du réceptacle et la diffusion de vapeur d'eau dans le biberon. Cette conformation permet d'introduire le col (B1) du biberon, de sorte qu'une partie avant (B2) de celui-ci vient en débordement extérieur sous la grille et l'autre (B3) en butée d'appui contre le fond (5.7) de la bouche. L'assise (5.1) présente une incurvation (5.8) transversale permettant une bonne tenue du biberon en enveloppant partiellement le corps du biberon.

Ainsi le moyen (5) tel que décrit est de nature à éviter, lors de manipulations malencontreuses de l'appareil stérilisateur, tout échappement du biberon par rapport à la grille et son basculement latéral. Les différents moyens (5) disposés sur la grille sont avantageusement conformés monobloc avec celle-ci.

Dans une variante de réalisation, la forme (5.3) de tenue du col du biberon est simplement établie selon une bride (5.10). Il y a lieu de noter que les formes en bouche ou de la bride sont établies dimensionnellement pour permettre sans aucune difficulté la tenue des biberons dont les cols (B1) peuvent sensiblement varier en diamètre pour permettre une utilisation à la plupart des types de biberons. La partie bride ou le plan extérieur de la bouche peut venir alors en appui direct contre la partie épaulée (B4) formée entre le corps (B5) du biberon et le col (B1).

Ainsi qu'il apparaît aux dessins, la bouche peut présenter intérieurement une forme ovalisée ou en variante sphérique. Elle peut être agencée avec des saillies intérieures espacées (5.9) pouvant correspondre au filet de vissage établi sur le col du biberon pour la fixation du capuchon correspondant. Les saillies contribuent à assurer la stabilité du biberon en faisant un contre-appui sur la partie col.

Selon une autre caractéristique de l'invention, la disposition des différentes moyens (5) sur la grille peut être réalisée par exemple dans la configuration illustrée aux figures 1 à 3. Dans ce cas, la grille est aménagée avec trois moyens (5) qui sont orientés angulairement selon une triangulation permettant de manière très caractéristique le positionnement des biberons sans qu'il y ait un quelconque appui ou entre-choquement des biberons entre eux.

Ainsi qu'il apparaît aux figures 1 à 3 des dessins, la partie arrière du corps d'un biberon vient sensiblement dans un plan au-dessus de la partie avant du corps du biberon adjacent. Cette disposition en triangulation équilatérale permet ainsi un rangement optimisé des biberons, de leurs tétines et couvercles dans l'appareil en vue de la stérilisation. Le degré d'inclinaison des assises (5.1) est tel qu'il n'y a aucun contact entre les corps de biberons, tout en limitant au maximum la hauteur de l'ensemble de l'appareil pour être introduit dans un four à micro-ondes.

Selon les volumes acceptables de ceux-ci, la grille de l'appareil peut être aménagée en variante avec deux ensembles de trois biberons disposés par couple en parallèle selon la configuration en triangulation.

Dans la variante de la figure 5, les moyens (5) sont disposés en ligne, ce qui permet par exemple d'obtenir un appareil stérilisateur de configuration carrée ou rectangulaire. Les trois moyens peuvent être disposés dans le même sens ou en alternance. L'inclinaison des biberons et les dimensions de la grille sont établies pour permettre le rangement des biberons, des tétines et des couvercles.

Les avantages ressortent bien de l'invention. On souligne en particulier la meilleure stabilité et tenue des biberons La vapeur d'eau est diffusée très rapidement dans le volume intérieur de l'appareil à travers la pluralité d'ouvertures formées sur la grille en permettant une stérilisation des différents composants. Très avantageusement, cette vapeur est guidée par les bouches à l'intérieur de chacun des biberons pour assurer une parfaite stérilisation.

On souligne également la plus grande rapidité de stérilisation des biberons et en particulier à l'intérieur de ceux-ci.

## Revendications

1. Appareil pour maintien de biberons en vue de leur stérilisation du type comprenant un réceptacle de fond (2) susceptible de recevoir un volume d'eau, ledit réceptacle (2) recevant à une hauteur déterminée une grille (3) monobloc avec une pluralité de découpes (3.1) agencée avec des moyens (5) permettant l'engagement et le positionnement des biberons par leur col dans une position inclinée par rapport à la verticale, ledit réceptacle recevant une cloche d'obturation (4), l'appareil pouvant être introduit dans un four à micro-ondes, ladite grille (3) permettant le positionnement des biberons, tétines et capuchons,
caractérisé en ce que le réceptacle (2) présente, dans son fond, un ou des bandeaux supports (2.1) de la grille (3), ladite grille (3) étant munie d'une pluralité desdits moyens (5) permettant l'engagement et le positionnement des biberons, ces moyens (5) étant conformés et agencés pour assurer, en même temps, la tenue de chaque biberon par son col et son maintien par son corps, chacun desdits moyens (5) étant agencé avec une assise (5.1) profilée présentant un plan (5.2) incliné et une incurvation (5.8) transversale enveloppant partiellement le corps du biberon, cette assise (5.1) se prolongeant dans sa partie basse avec une forme (5.3) en collet susceptible d'entourer le col du biberon, ladite forme (5.3) présentant une ouverture (5.5) de passage du col du biberon perpendiculaire au plan de l'assise (5.1).

2. Appareil stérilisateur selon la revendication 1, caractérisé en ce que l'assise (5.1) de chaque moyen (5) est profilée selon une conformation triangulaire avec une base dans le plan horizontal de la grille.

3. Appareil stérilisateur selon les revendications 1 et 2, caractérisé en ce que la forme en collet (5.3) est agencée avec une bouche (5.4) ayant une ouverture (5.5) de passage du col du biberon, ladite bouche se prolongeant avec une partie pleine coudée (5.6) reliée au plan horizontal de la grille, tandis qu'une découpe (3.4) est formée sur la grille à l'intérieur de la bouche pour permettre une communication avec la partie de fond du réceptacle et l'accès de vapeur d'eau dans le biberon.

4. Appareil stérilisateur selon la revendication 3, caractérisé en ce que la bouche présente intérieurement une pluralité de saillies espacées (5.9) correspondant au filet de vissage établi sur le col du biberon, lesdites saillies contribuant à assurer la stabilité du biberon en faisant contre-appui sur la partie col.

5. Appareil stérilisateur selon les revendications 1 et 2, caractérisé en ce que la forme en collet (5.3) de tenue du col du biberon est une bride (5.10).

6. Appareil stérilisateur selon l'une quelconque des revendications 1 à 5, caractérisé en ce que les moyens (5) permettant l'engagement et le positionnement des biberons sont au nombre de trois et orientés angulairement en triangulation équilatérale selon une disposition où la partie arrière du corps d'un biberon vient sensiblement dans un plan au-dessus de la partie avant du corps du biberon adjacent, le degré d'inclinaison des assises (5.1) étant établi pour éviter tout contact des biberons entre eux.

7. Appareil stérilisateur selon l'une quelconque des revendications 1 à 5, caractérisé en ce que les moyens (5) permettant l'engagement et le positionnement des biberons sont disposés en linéaire dans le même sens ou en alternance de positions.

8. Appareil stérilisateur selon la revendication 6, caractérisé en ce que les moyens (5) permettant l'engagement et le positionnement des biberons sont tels que deux ensembles de trois biberons disposés par couple en parallèles selon une configuration en triangle peuvent prendre place dans l'appareil.

## Claims

1. An appliance for holding baby milk bottles for the purpose of their sterilisation of a type comprising a bottom container (2) capable of enclosing a volume of water, the aforementioned container (2) receiving at a set height an integrally formed grid (3) provided with a plurality of cut-outs (3.1) equipped with means (5) allowing the engagement and positioning of baby milk bottles by their neck in an inclined position with respect to vertical, the aforementioned container receiving a bell cover (4), the appliance being capable of placement in a microwave oven, the aforementioned grid (3) allowing the positioning of baby milk bottles, teats and caps, characterised in that the bottom of the container (2) features one or more ribs (2.1) for supporting the grid (3), the aforementioned grid (3) being provided with a plurality of the aforementioned means (5) allowing the engagement and positioning of baby milk bottles, these means (5) being formed and equipped to ensure simultaneously that each baby milk bottle is held by its neck and supported by its body, each one of the aforementioned means (5) being equipped with a shaped saddle (5.1) providing an inclined surface (5.2) and a transverse concavity (5.8) partially enclosing the body of the baby milk bottle, this saddle (5.1) being extended at its lower end into a collar form (5.3) capable of encircling the neck of the baby milk bottle, the aforementioned collar form (5.3) featuring an opening (5.5) perpendicular to the surface of the saddle (5.1) for inserting the neck of the baby milk bottle.

2. A sterilisation appliance according to claim 1, characterised in that the saddle (5.1) of each means (5) is shaped according to a triangular formation with its base lying in the horizontal plane of the grid.

3. A sterilisation appliance according to claims 1 and 2, characterised in that the collar form (5.3) is fitted with an inlet port (5.4) with an opening (5.5) for passing the neck of the baby milk bottle, the aforementioned inlet port being extended into a continuous elbow section (5.6) connected to the horizontal surface of the grid, whilst a cut-out (3.4) is made in the grid inside the inlet port to provide communication with the bottom section of the container and a means of access for water vapour to enter the baby milk bottle.

4. A sterilisation appliance according to claim 3, characterised in that the interior of the inlet port features a plurality of projections (5.9) spaced according to the screw thread incorporated on the baby milk bottle neck, the aforementioned projections contributing to ensure the stability of the baby milk bottle by exerting a counter-pressure onto its neck section.

5. A sterilisation appliance according to claims 1 and 2, characterised in that the collar form (5.3) holding the baby milk bottle neck is a flange (5.10).

6. A sterilisation appliance according to any one of claims 1 to 5, characterised in that the means (5) allowing engagement and positioning of baby milk bottles are three in number and are oriented anglewise in equilateral triangulation according to an arrangement in which the rear section of one baby milk bottle lies approximately in a plane above the front section of the adjacent baby milk bottle body, the degree of inclination of the saddles (5.1) being established to prevent any mutual contact of the baby milk bottles.

7. A sterilisation appliance according to any one of claims 1 to 5, characterised in that the means (5) allowing engagement and positioning of baby milk bottles are linearly arranged in the same direction or in alternating positions.

8. A sterilisation appliance according to claim 6, characterised in that the means (5) allowing engagement and positioning of baby milk bottles are such that two sets of three baby milk bottles arranged in parallel pairs according to a triangular configuration can be placed in the appliance.

## Patentansprüche

1. Gerät für das Halten von Babyflaschen zwecks Sterilisierung mit einem Bodenbehälter (2), der geeignet ist, eine bestimmte Wassermenge aufzunehmen, wobei der Behälter (2) in einer festgelegten Höhe einen einstückigen Rost (3) mit mehreren Ausschnitten (3.1) aufnimmt, der Einrichtungen (5) besitzt, die das halsseitige Einführen und Positionieren der Babyflaschen in einer gegenüber der Senkrechten geneigten Lage ermöglichen, wobei der Behälter mit einer Glocke (4) verschlossen wird, das Gerät in einen Mikrowellenherd gestellt werden kann und der Rost (3) die Lagerung von Babyflaschen, Sauger und Verschlußkappen ermöglicht,
dadurch gekennzeichnet, daß der Behälter (2) am Boden ein oder mehrere Trageleisten (2.1) für den Rost (3) aufweist, wobei der Rost (3) mit mehreren Einrichtungen (5) versehen ist, die das Einführen und Positionieren der Babyflaschen ermöglichen und die so ausgebildet und beschaffen sind, daß sie gleichzeitig die Fixierung jeder einzelnen Babyflasche am Hals und den Halt des Flaschenkörpers gewährleisten, wobei jede dieser Einrichtungen (5) mit einem profilierten Auflageteil (5.1) ausgebildet ist, das eine schräge Ebene (5.2) und eine querverlaufende Einwärtskrümmung (5.8) aufweist, die den Flaschenkörper teilweise umhüllt, wobei das Auflageteil (5.1) im unteren Teil durch eine Ringform (5.3) verlängert wird, die geeignet ist, den Hals der Babyflasche zu umgeben, wobei die Form (5.3) senkrecht zur Ebene des Auflageteils (5.1) eine Öffnung (5.5) für die Durchführung des Flaschenhalses aufweist.

2. Sterilisiergerät nach Anspruch 1, dadurch gekennzeichnet, daß das Auflageteil (5.1) jeder Einrichtung (5) mit einem Dreiecksprofil ausgebildet ist, wobei die Basis in der waagrechten Ebene des Rostes verläuft.

3. Sterilisiergerät nach den Ansprüchen 1 und 2, dadurch gekennzeichnet, daß die Ringform (5.3) mit einer Mündung (5.4) beschaffen ist, die eine Öffnung (5.5) für die Durchführung des Flaschenhalses aufweist, wobei die Mündung durch ein abgewinkeltes Vollmaterialteil (5.6) verlängert wird, das mit der waagrechten Ebene des Rostes verbunden ist, während innerhalb der Mündung am Rost ein Ausschnitt (3.4) ausgebildet ist, um eine Verbindung mit dem Bodenteil des Behälters und den Zustrom von Wasserdampf in die Babyflasche zu ermöglichen.

4. Sterilisiergerät nach Anspruch 3, dadurch gekennzeichnet, daß die Mündung innen mehrere in Abständen angeordnete Vorsprünge (5.9) aufweist, die dem Schraubgewinde am Flaschenhals entsprechen, wobei diese Vorsprünge dazu beitragen, durch Gegenlagerfunktion am Halsteil die Stabilität der Babyflasche zu gewährleisten.

5. Sterilisiergerät nach den Ansprüchen 1 und 2, dadurch gekennzeichnet, daß es sich bei der Ringform (5.3) für den Halt des Flaschenhalses um einen Flansch (5.10) handelt.

6. Sterilisiergerät nach einem der Ansprüche 1-5, dadurch gekennzeichnet, daß drei Einrichtungen (5) für das Einführen und Positionieren der Babyflaschen vorgesehen sind, die in gleichzeitiger Triangulation nach einer Anordnung winkelig ausgerichtet sind, bei der der hintere Teil des Körpers einer Babyflasche ziemlich genau in einer Ebene oberhalb des Vorderteils des Körpers der benachbarten Flasche zu liegen kommt, wobei der Neigungsgrad der Auflageteile (5.1) so beschaffen ist, daß jeglicher Kontakt der Babyflaschen untereinander vermieden wird.

7. Sterilisiergerät nach einem der Ansprüche 1-5, dadurch gekennzeichnet, daß die Einrichtungen (5) für das Einführen und Positionieren der Babyflaschen linear in gleicher Richtung oder in alternierender Position angeordnet sind.

8. Sterilisiergerät nach Anspruch 6, dadurch gekennzeichnet, daß die Einrichtungen (5) für das Einführen und Positionieren der Babyflaschen so beschaffen sind, daß zwei Gruppen aus drei paarweise parallel angeordneten Babyflaschen in einer Dreieckskonfiguration im Gerät Platz finden.
